# EUROPEAN PATENT SPECIFICATION

(11) **EP 1 133 459 B1**
(45) Date of publication and mention of the grant of the patent: **11.01.2006**
(21) Application number: 99959091.2
(22) Date of filing: 24.11.1999
(51) Int. Cl.: C07C 1/00, C07C 211/42, C07C 209/84

(54) **SERTRALINE HYDROCHLORIDE FORM V**
SERTRALIN HYDROCHLORID FORM V
CHLORHYDRATE DE SERTRALINE FORME V

(30) Priority: 27.11.1998 US 110113 P; 19.03.1999 US 125172 P; 07.05.1999 US 133117 P; 09.08.1999 US 147888 P
(43) Date of publication of application: 19.09.2001
(60) Divisional application: 05024394.8
(73) Proprietor: TEVA PHARMACEUTICAL INDUSTRIES LTD., 49131 Petah Tiqva (IL)
(72) Inventor: SCHWARTZ, Eduard, Rechovot (IL); NIDAM, Tamar, Yehud (IL); LIBERMAN, Anita, Tel-Aviv (IL); MENDELOVICI, Marioara, Rechovot (IL); ARONHIME, Jehudit, 44242 Kfar Saba (IL); SINGER, Claude, Kfar Saba (IL); VALDMAN, Evgeni, Petah Tikva (IL)
(74) Representative: Nachshen, Neil Jacob
(86) International application number: PCT/US1999/027881
(87) International publication number: WO 2000/032551

(56) References cited:
- WO-A-01/32601
- US-A- 4 536 518
- US-A- 5 082 970
- US-A- 5 248 699
- US-A- 5 463 126
- US-A- 5 734 083
- PATENT ABSTRACTS OF JAPAN vol. 2000, no. 04, 31 August 2000 (2000-08-31) & JP 2000 026378 A (SUMIKA FINE CHEMICALS CO LTD), 25 January 2000 (2000-01-25)

## Description

### FIELD OF THE INVENTION

The present invention relates to a novel, reproducible method for preparing previously reported polymorph Form V crystalline sertraline hydrochloride, (1S-cis)-4-(3,4-dichlorophenyl)-1,2,3,4-tetrahydro-N-methyl-1-naphthalenaminehydrochloride.

### BACKGROUND OF THE INVENTION

Sertraline hydrochloride, (1S-cis)-4-(3,4 dichlorophenyl)-1,2,3,4-tetrahydro-N-methyl-1-naphthalenamine hydrochloride, having the formula 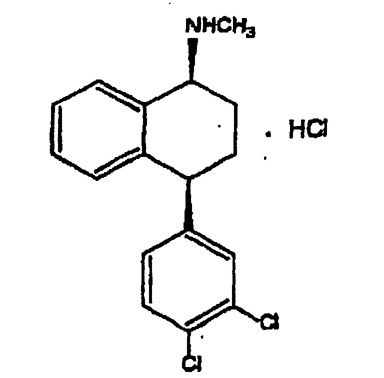 is approved, under the trademark Zoloft®, by the U.S. Food and Drug Administration, for the treatment of depression, obsessive-compulsive disorder and panic disorder.

U.S. Patent No. 4,536,518 describes a synthesis of sertraline hydrochloride. U.S. Patent No. 5,248,699 describes five crystalline forms of sertraline hydrochloride, designated Form I, Form II, Form III, Form IV and Form V.

U.S. Patent 4,536,518 ("the '518 patent") describes the preparation of sertraline hydrochloride with a melting point of 243-245°C by treating an ethyl acetate/ether solution of the free base with gaseous hydrogen chloride. The solid state properties of the sertraline hydrochloride so produced are not otherwise disclosed.

According to U.S. Patent No. 5,248,699 ("the '699 patent"), the sertraline hydrochloride produced by the method of the '518 patent has a crystalline form denominated "Form II". The '699 patent discloses four other polymorphs I, III, IV, and V, and characterizes them by single crystal x-ray analysis, powder x-ray diffraction, infra-red spectroscopy, and differential scanning calorimetry. The '699 patent reports that Form II is produced by rapid crystallization of sertraline hydrochloride from an organic solvent, including isopropyl alcohol, hexane and generally describes methods for making sertraline hydrochloride Forms I-V. According to this patent, the preferential formation of Forms I, II or IV in an acidic solution consisting of isopropyl alcohol, hexane, acetone, methyl isobutyl ketone, glacial acetic acid, or preferably, ethyl acetate depends on the rapidity of crystallization. Form I is described as being made by crystallizing sertraline hydrochloride in an acidic solution using an organic solvent such as those listed above. The crystallization of Form I is carried out at a temperature from about 20°C to about the solvent reflux temperature, preferably from about 40° to 60°C. The only method described in this patent for making Forms II and IV is by the rapid crystallization of sertraline hydrochloride from an organic solvent such as those listed above. Slow crystallization or granulation of sertraline hydrochloride will produce Form L Form III is described as being formed by heating Forms I, II or IV to temperatures above about 180° C. Granulating either of Forms II, III or IV in isopropyl alcohol, ethyl acetate, hexane or any of the solvents listed above at a temperature from about 40° to 60° C causes conversion of Form I. The only method described in this patent for making Form V is by sublimation of sertraline hydrochloride Form I at reduced pressure and at a temperature from about 180-190° C onto a condenser. However in our hands attempts to repeat this procedure to obtain Form V have been unsuccessful.

US 5 734 083 discloses a process for preparing the T1 polymorph of sertraline hydrochloride. In this process, a slurry or solution of sertraline free base in an organic nonpolar solvent such as toluene is treated with a polar solvent such a ethyl acetate, diethyl ether or mixtures thereof, so as to form a solution of the sertraline free base in the polar solvent. A separate batch of the organic solvent is taken and cooled, e.g. to 0°-10° C., and hydrogen chloride gas is passed into it, to prepare a solution which is about 1-10% (w/w) in hydrogen chloride then added to the sertraline free base solution slowly, with moderate agitation.

### SUMMARY OF THE INVENTION

It has now been discovered that sertraline hydrochloride Form V can be formed by crystallization from various solvents rather than by sublimation. In particular, the present invention relates to a process for making sertraline hydrochloride Form V comprising the steps of:
(a) dissolving or suspending sertraline base in a solvent selected from the group consisting of methanol, ethanol, water, isopropyl alcohol, hexane and toluene and mixtures thereof;
(b) adding hydrogen chloride or hydrochloric acid to reduce the pH of the solution or suspension; and
(c) isolating sertraline hydrochloride Form V.

There is also provided a new crystal form of sertraline hydrochloride, denominated Form VL Form VI is useful as an intermediate in one aspect of the present invention.

### BRIEF DESCRIPTION OF THE DRAWINGS

Figure 1 is a characteristic x-ray powder diffraction spectrum of sertraline hydrochloride Form V.
Figure 2 is a characteristic x-ray powder diffraction spectrum of amorphous sertraline hydrochloride.
Figure 3 is a characteristic differential scanning calorimetric (DSC) thermogram of sertraline hydrochloride Form V.
Figure 4 is a characteristic infrared (IR) absorption spectrum of sertraline hydrochloride Form V.
Figure 5 is a characteristic differential scanning calorimetric (DSC) thermogram of sertraline hydrochloride Form VI.

### DETAILED DESCRIPTION OF THE INVENTION

### Form V

The present invention provides a new process for making sertraline hydrochloride Form V from sertraline base. The method provided in the present invention is more commercially practicable than the sublimation-condensation method of U.S. Patent No. 5,249,699, which we have not been able to reproduce. It has also surprisingly been found that, by the present method, Form V is formed even at different crystallization rates.

Where the present invention provides methods for the conversion of sertraline hydrochloride to sertraline hydrochloride Form V, sertraline hydrochloride is combined with a solvent selected from the group consisting of methanol, ethanol, 1-methoxy-2-propanol and a mixture of isopropyl alcohol and water. If a mixture of isopropyl alcohol and water is used, it is preferably an about 6:1 mixture. Preferably the solvent is methanol or ethanol, and most preferably the solvent is ethanol. Sertraline hydrochloride Form V is isolated by allowing the solution to cool. One preferred method is to rapidly cool the solvent to 5°C. Another preferred method comprises seeding the solution with sertraline hydrochloride Form V crystals, followed by slow cooling to room temperature, followe by filtration and drying. Sertraline hydrochloride Form V can also be prepared by recrystallizing sertraline hydrochloride Form VI (described below) from water.

The present invention provides methods for the conversion of sertraline base to sertraline hydrochloride Form V, wherein sertraline base is added to at least one solvent, and hydrogen chloride gas is bubbled through the solution. Preferred solvents include methanol, ethanol, water, or mixtures of ethanol, methanol, isopropyl alcohol, hexane with each other or with water.

Alternatively, an appropriate amount of hydrogen chloride gas dissolved in methanol, ethanol, water, isopropyl alcohol, ethyl acetate, or a mixture thereof is combined with the sertraline base solution. Sertraline hydrochloride Form V is isolated by allowing precipitation to occur from about 0° to about 60°C followed by filtration and drying. Preferably the solvent is hexane, isopropyl alcohol or a mixture thereof. In another method, sertraline base is added to a solvent and the resulting solution is added to a hydrochloric acid solution of pH 0-4; preferably the pH of the solution is about 1.

Alternatively, sertraline base is added to a solvent selected from the group consisting of methanol, ethanol, water, hexane and isopropyl alcohol or a mixture thereof. The solution is heated and concentrated hydrochloric acid is added. Water may also be added. Sertraline hydrochloride Form V is isolated by allowing the mixture to cool to room temperature and remain at room temperature overnight, followed by filtration and drying.

Alternatively, sertraline base may be combined with a solvent selected from the group consisting of ethanol or ethanol and water. The solution is heated to about 50-60°C and water is added. The solvent is partially removed by distillation. Sertraline hydrochloride Form V is isolated by allowing the solution to cool to room temperature, followed by filoratian and drying at the precipitate.

Alternatively, sertraline base may be combined with a solvent selected from the group consisting of methanol ethanol and a mixture thereof. A saturated solution of hydrogen chloride gas in isopropyl alcohol is added to induce formation of seirtraline hydrochloride Form V. Sertraline hydrochloride Form V is isolated by allowing the solution to stand at room temperature overnight, followed by filtration and drying of the precipitate.

Sertraline base for use in the processes of the present invention may be produced by dissolving sertraline mandelate in ethyl acetate followed by neutralization of the sertraline mandelate with aqueous sodium hydroxide. The organic phase is separated from the aqueous phase and dried using magnesium sulfate. The solvent is removed under reduced pressure to produce sertraline base as an oil. Methods for making sertraline base are set forth in U.S. Patent Nos. 4,536,518 and 5,249,699.

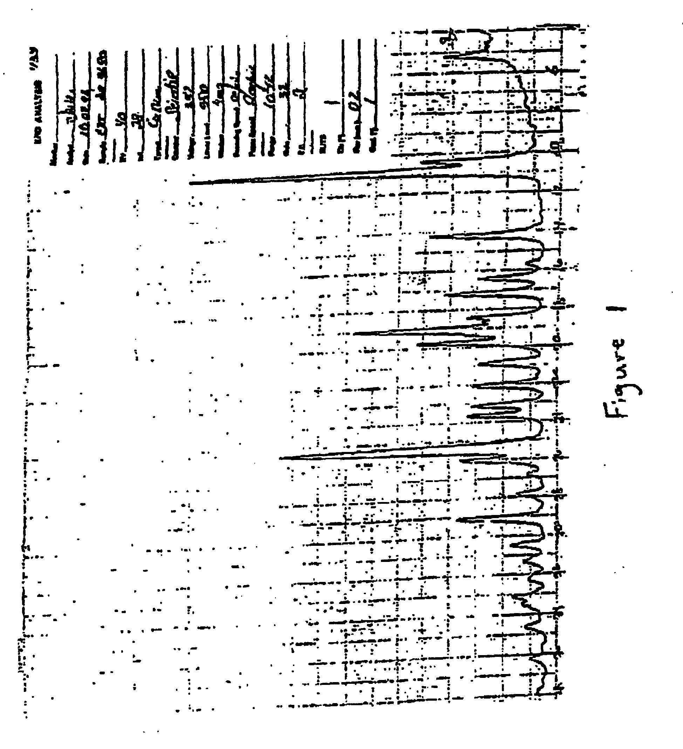

The sertraline hydrochloride Form V that results from practicing the invention as exemplified herein can be characterized by its powder X-ray diffraction pattern. Figure 1 is a representative pattern of sertraline hydrochloride Form V. The principal peaks observed are at about 5.2° ±0.2, 10.4° ±0.2, 11.0° ±0.2, 14.3° ±0.2, 16.5° ±0.2, 17.3° ±0.2, 18.4° ±0.2, 19.1°±0.2, 19.7° ±0.2, 20.9° ±0.2, 22.0° ±0.2, 23.2° ±0.2, 23.6° ±0.2, 25.5° ±0.2, 26.0°±0.2, and 29.1° ±0.2 degrees 2 theta.

Three experiments were performed in order to repeat the procedure described in U.S. Patent No. 5,248,699 for preparing Form V by sublimation. Two experiments were performed by sublimating a sample of Form I under 30 mm Hg vacuum and temperature between 170-190°C. A third experiment was performed by sublimating a sample of Form I under high vacuum (13.33 Pa (0.1 mm Hg)) and temperature between 180-195°C.

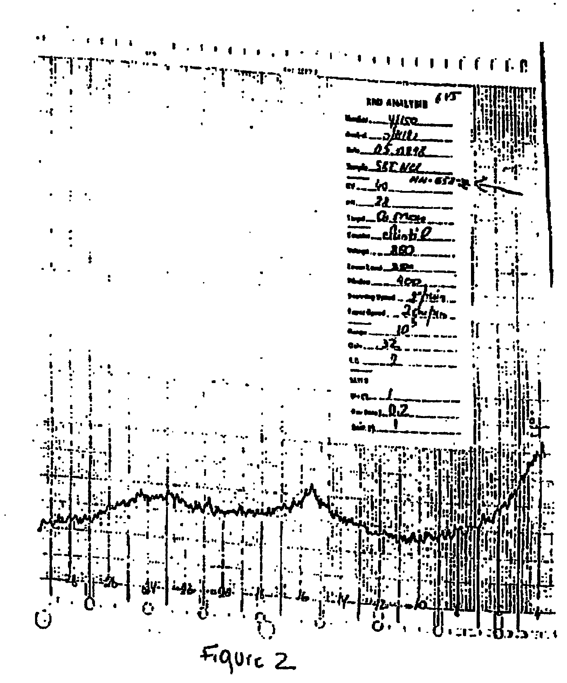

The three samples of sertraline hydrochloride prepared by sublimation were analyzed by powder x-ray diffraction. In all cases, the typical broad featureless pattern without sharp peaks typical of amorphous materials was obtained. Figure 2 is one such pattern.

In conclusion, sertraline hydrochloride could not be obtained by following the procedure set forth in U.S. Patent No. 5,248,699 for preparing Form V by sublimation of Form L

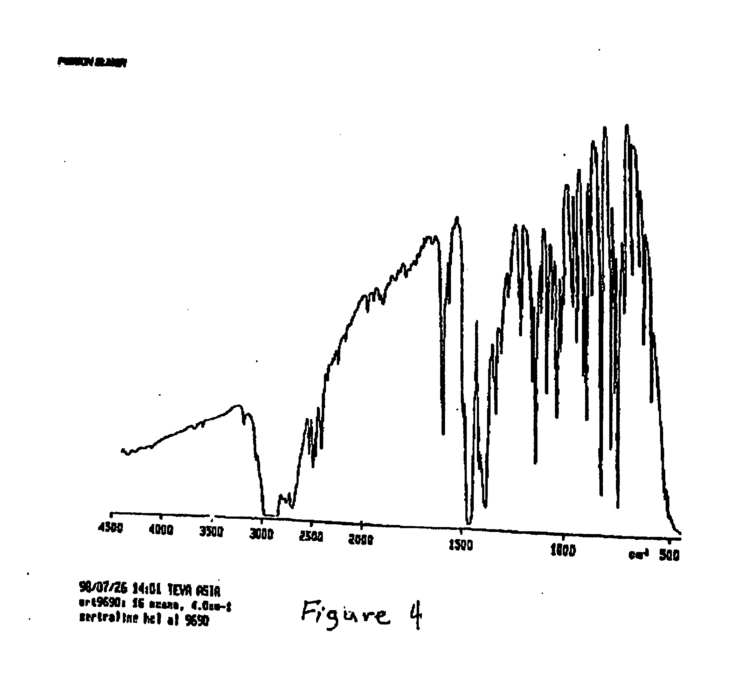

The IR spectrum of sertraline hydrochloride Form V produced by the present process is characterized by the following bands: 773 cm⁻¹, 822 cm⁻¹, cm⁻¹, 1012 cm⁻¹, 1032 cm⁻¹, 1054 cm⁻¹, 1133 cm⁻¹, 1328 cm⁻¹, 1562 cm⁻¹, and 1590 cm⁻¹, as shown in Figure 4.

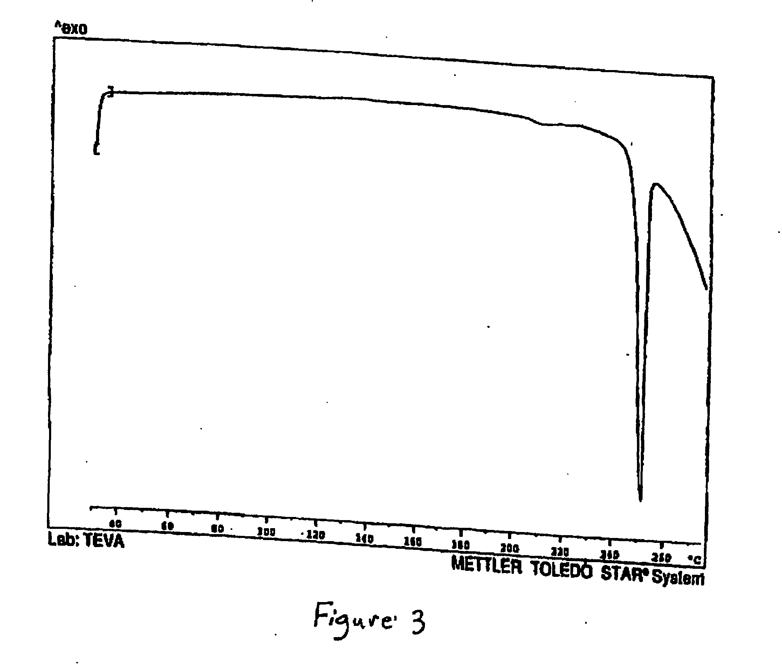

The sertraline hydrochloride Form V of the present process is further characterized by the DSC thermogram data, for example, as disclosed in Figure 3. The DSC thermogram is characterized by a small endotherm (-3 Joule per gram) at about 210°C, believed to be a solid-solid transformation (based upon observation under a hot stage microscope) to Form III, and a melting peak. Form III at 251°C

### Form VI

Sertraline hydrochloride Form VI is a solvated crystal form of sertraline hydrochloride In the present invention, sertraline hydrochloride Form VI may be an ethanolate, wherein ethanol is Incorporated into the crystal structure of Form VI. Alternatively, sertraline hydrachlorida Form VI may be a methanolate, wherein methanol is incorporated into the crystal structure of sertraline hydrochloride Form VI. All sertraline hydrochloride Form VI solvates have identical powder x-ray diffraction patterns. Therefore, when referring to sertraline hydrochloride Form VI all sertraline hydrochloride Form VI solvates such as, sertraline hydrochloride Form VI ethanolate and sertraline hydrochloride Form VI methanolate, are necessarily included.

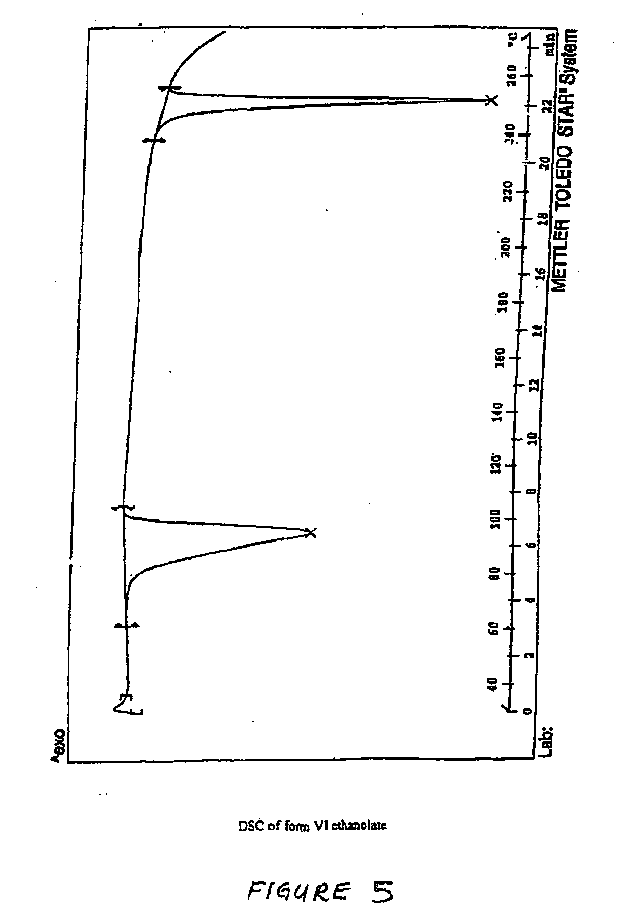

To form the novel crystalline form sertraline hydrochloride Form VI, sertraline base is added to absolute ethanol or methanol. Hydrogen chloride gas is then bubbled through the solution. Sertraline hydrochloride Form VI is isolated by allowing precipitation to occur, followed by filtration. The DSC thermogram of Form VI crystallized from ethanol displays a desolvation peak at 95°C (see Fig. 5) and loses 11.2% weight (by TGA); Form VI crystallized from methanol loses 8.3 % weight (by TGA) upon desolvation; Form VI crystallized from ethanol is an ethanolate, and more specifically is a monoethanolate. Form VI crystallized from methanol is a methanolate, and more specifically is a monomethanolate.

Drying of the precipitated sertraline hydrochloride Form VI at 50-60°C overnight yields sertraline hydrochloride Form V.

### Pharmaceutical Compositions Containing Sertraline Hydrochloride Polymorphs

In accordance with the present invention,
Form V of sertraline hydrochloride prepared by the new methods disclosed herein may be prepared as pharmaceutical compositions that are particularly useful for the treatment of depression, obsessive-compulsive disorder and panic disorder. Such compositions comprise one of the new crystalline forms of sertraline hydrochloride with pharmaceutically acceptable carriers and/or excipients known to one of skill in the art.

For example, these compositions may be prepared as medicaments to be administered orally, parenterally, rectally, transdermally, bucally, or nasally. Suitable forms for oral administration include tablets, compressed or coated pills, dragees, sachets, hard or gelatin capsules, sub-lingual tablets, syrups and suspensions. Suitable forms of parenteral administration include an aqueous or nonaqueous solution or emulsion, while for rectal administration suitable forms for administration include suppositories with hydrophilic or hydrophobic vehicle. For topical administration the invention provides suitable transdermal delivery systems known in the art, and for nasal delivery there are provided suitable aerosol delivery systems known in the art.

### Experimental

The powder X-ray diffraction patterns were obtained by methods known in the art using a Philips X-ray powder diffractometer, Goniometer model 1050/70 at a scanning speed of 2° per minute, with a Cu radiation of λ = 1.5418 A

The differential scanning calorimeter thermograms were obtained by methods known in the art using a DSC Mettler 821 Star°. The weight of the samples was less than 5 mg. The temperature range of scans was 30°C-300°C at a rate of 10°C/min. Samples were purged with nitrogen gas at a flow rate of 40 mL/min. Standard 40 µl aluminum crucibles were used having lids with three small holes.

The infrared spectra were obtained by methods known in the art using a Perkin Elmer FT-IR Paragon 1000 spectrometer. Samples were analyzed in Nujol mulls. Spectra were obtained at 4 cm⁻¹ resolution and 16 scans each.

### EXAMPLES

The present invention will now be further explained in the following examples. However, the present invention should not be construed as limited thereby. One of ordinary skill in the art will understand how to vary the exemplified preparations to obtain the desired results.

### Example 1

### Preparation of Sertraline Base

Sertraline mandelate (5 g) was stirred at room temperature with 50 mL ethyl acetate. Aqueous sodium hydroxide was added dropwise until the sertraline mandelate was completely neutralized. The phases were separated and the organic phase was dried over MgSO₄ and filtered. The solvent was removed under reduced pressure resulting sertraline base as an oil (3.2 g).

### Example 2

### Preparation of Sertraline Hydrochloride Form VI and Form V

Sertraline base (25 g) was dissolved in methanol (125 mL) at room temperature. The solution was acidified with hydrogen chloride until pH 1.5 was reached. (Precipitation occurred during acidification.) The temperature rose to approximately 40°C. The slurry was allowed to cool to room temperature and stirred for about 2 hours. The solid was separated by filtration to give sertraline hydrochloride methanolate-Form VI. Drying the product overnight gave sertraline hydrochloride Form V.

### Example 3

### Preparation of Sertraline Hydrochloride Form VI and Form V

Sertraline base (3.2 g) was dissolved in absolute ethanol (32 mL) at room temperature and then hydrogen chloride gas was bubbled in until pH 0.5 was reached. The temperature rose to 40°C. The slurry was allowed to cool to room temperature and stirred for about 16 hours. The solid was separated by filtration, and washed with ethanol (3 x 2 mL). Figure 5 sets forth the X-ray diffraction pattern of the product (sertraline hydrochloride Form VI) so obtained. Drying overnight at 50-60°C of that product yields 2.95 g (82%) of sertraline hydrochloride Form V.

### Example 4

### Preparation of Sertraline Hydrochloride Form V

Sertraline base (3 g) was dissolved in absolute ethanol (15 mL) at room temperature. A saturated solution of hydrogen chloride in isopropyl alcohol was added dropwise to reach a pH of 1.3. The resulting slurry was stirred at room temperature overnight. The solid was separated by filtration and dried overnight at 50-60°C yielding 2.75 g (81. 8%) sertraline hydrochloride Form V.

### Example 5

### Preparation of Sertraline Hydrochloride Form V

Sertraline base (3 g) was dissolved in absolute ethanol (15.5 mL) at room temperature and then the solution was cooled to approximately 0°C. Hydrogen chloride gas was bubbled until pH 0.5 was reached. The temperature rose to approximately 7°C. Precipitation occurred and the slurry was stirred at about 10°C for 2 hours. The solid was isolated by filtration, washed with ethanol and dried at approximately 50°C. The dried material (2.87 g, yield 82.7%) was sertraline hydrochloride Form V.

### Example 6

### Preparation of Sertraline Hydrochloride Form V

Sertraline base (3 g) was stirred with 35 mL water. The slurry was heated at -70°C and, while maintaining this temperature, concentrated hydrochloric acid was added until pH 1 was reached. During acidification, almost complete dissolution was observed followed by precipitation. The mixture was cooled to room temperature and stirred for 2 hours. The solid was isolated by filtration, washed with water and dried overnight at 50-60° C, yielding 3.23 g (96%) sertraline hydrochloride Form V.

### Example 7

### Preparation of Sertraline Hydrochloride Form V

Sertraline base (3 g) was dissolved in 10 mL absolute ethanol at 40°C. The solution was heated to 50°-60°C and concentrated hydrochloric acid 32% (1.2 mL) was added until pH ~ 1.3 was reached. Water (12 mL) was added. The resulting clear solution was concentrated to half its volume and was allowed to cool naturally to room temperature. The solid was isolated by filtration, washed with water and dried overnight at 50-60°C, yielding 3.18 g (94.65%) sertraline hydrochloride Form V.

### Example 8

### Preparation of Sertraline Hydrochloride Form V

Sertraline base (3.7 g) was dissolved in 18.5 mL absolute ethanol and the solution was heated to 60° C. Hydrogen chloride gas was bubbled through the ethanol solution until pH ~0.5 was reached. The mixture was cooled to room temperature and the stirring was continued for 2 hours. The solid obtained after filtration, washing with ethanol and drying at 50° C was sertraline hydrochloride Form V (3.16 g, yield 76%).

### Example 9

### Preparation of Sertraline Hydrochloride Form V

Sertraline free base was dissolved in ethanol absolute and the solution was acidified with hydrogen chloride gas to about pH 3. Precipitation occurs and the slurry was stirred at room temperature for 2 hours. The resulting solid was filtered, washed with ethanol and dried to yield sertraline hydrochloride Form V.

### Example 10

### Preparation of Sertraline Hydrochloride Form V

Sertraline free base (13.3 g) was dissolved in absolute ethanol (60 mL) and was added dropwise over one hour to ethanol (20 mL) containing hydrogen chloride gas (17.5 g) at 35° C. After 2 hours, the solid was filtrated, washed with ethanol and dried at about 80° C to yield sertraline hydrochloride Form V(12.9 g., yield 87%).

### Example 11

### Preparation of Sertraline Hydrochloride Form V

Anhydrous sertraline hydrochloride (2 g) was stirred with 14 mL absolute ethanol and heated to reflux to obtain a clear solution. The solution was seeded with sertraline hydrochloride Form V and cooled naturally to room temperature. Massive precipitation was observed at about 50° C. The slurry was stirred at room temperature during 2 hours. The solid was filtered, washed with ethanol (3 mL) and dried overnight at 50-60° C yielding 1.71 g (85.5%) of sertraline hydrochloride Form V.

Table 1 sets forth a summary of additional experiments conducted generally following procedures described above.

**TABLE I PREPARATION OF SERTRALINE NCL - FORM V SERTRALINE BASE AS STARTING MATERIAL**

| Exp't | Method of Crystallization | XRD | Yield |
|---|---|---|---|
| | | | (%) |
| A | Methanol/HCl gas | V | 78.7 |
| B | Methanol/HCl gas | V | 69 |
| C | Methanol/ HCl aqueous | V | 87.8 |
| D | Ethanol/HCl gas | V | 80.9 |
| E | Water/HCl aqueous | V | 96 |
| F | Hexane/Isopropyl alcohol/HCl gas | | |
| G | Methanol/HCl aqueous/water | V | 89 |
| H | Isopropyl alcohol/HCl aqueous/water | V | 78 |
| I | Ethanol/HCl aqueous/evaporation of ethanol | V | 96.1 |
| | | | |
| K | Ethanol/isopropyl alcohol/HCl gas | V | 81.8 |
| L | Methanol/isopropyl alcohol/HCl gas | V | 82.4 |
| | | | |
| | **SERTRALINE HCl AS STARTING MATERIAL** | | |
| M | Methanol (Form I and amorphous) | V | 60 |
| N | Ethanol (Form V) | V | 85.5 |
| O | Isopropyl alcohol/water (Form V) | V | 28 |

| | | | |
|---|---|---|---|
| PXRD = powder x-ray diffraction. | | | |

## Claims

1. A process for making sertraline hydrochloride Form V comprising the steps of:
(a) dissolving or suspending sertraline base in a. solvent selected from the group consisting of methanol, ethanol, water, isopropyl alcohol, hexane and toluene and mixtures thereof;
(b) adding hydrogen chloride or hydrochloric acid to reduce the pH of the solution or suspension; and
(c) isolating sertraline hydrochloride Form V.

2. The process of claim 1 wherein the pH of the solution or suspension of sertraline base and hydrogen chloride is 0 to 4.

3. The process of claim 1 wherein the solvent is methanol.

4. The process of claim 1 wherein the solvent is ethanol.

5. The process of claim 1 wherein the solvent is water.

6. The process of claim 1 wherein the solvent is a mixture of hexane and isopropyl alcohol.

7. The process of claim 1 wherein the solvent is a mixture of isopropyl alcohol and water.

8. The process of claim 1 wherein the solvent is a mixture of ethanol and water.

9. The process of claim 1 wherein the solvent is a mixture of ethanol and isopropyl alcohol.

10. The process of claim 1 wherein the solvent is a mixture of methanol and isopropyl alcohol.

11. A process for preparing a pharmaceutical composition comprising a therapeutically effective amount of sertraline hydrochloride Form V and a pharmaceutically acceptable carrier which process comprises the process of any one of the preceding claims.

## Patentansprüche

1. Verfahren zur Herstellung von Sertralin-Hydrochlorid Form V mit den Stufen, in denen man
(a) Sertralin-Base in einem Lösungsmittel löst oder suspendiert, welches aus der Gruppe ausgewählt ist, bestehend aus Methanol, Ethanol, Wasser, Isopropylalkohol, Hexan und Toluol und Gemischen davon,
(b) Chlorwasserstoff oder Chlorwasserstoffsäure hinzufügt, um den pH-Wert der Lösung oder Suspension zu erniedrigen, und
(c) Sertralin-Hydrochlorid Form V isoliert.

2. Verfahren nach Anspruch 1, wobei der pH-Wert der Lösung oder Suspension von Sertralin-Base und Chlorwasserstoff 0 bis 4 beträgt.

3. Verfahren nach Anspruch 1, wobei das Lösungsmittel Methanol ist.

4. Verfahren nach Anspruch 1, wobei das Lösungsmittel Ethanol ist.

5. Verfahren nach Anspruch 1, wobei das Lösungsmittel Wasser ist.

6. Verfahren nach Anspruch 1, wobei das Lösungsmittel ein Gemisch aus Hexan und Isopropylalkohol ist.

7. Verfahren nach Anspruch 1, wobei das Lösungsmittel ein Gemisch aus Isopropylalkohol und Wasser ist.

8. Verfahren nach Anspruch 1, wobei das Lösungsmittel ein Gemisch aus Ethanol und Wasser ist.

9. Verfahren nach Anspruch 1, wobei das Lösungsmittel ein Gemisch aus Ethanol und Isopropylalkohol ist.

10. Verfahren nach Anspruch 1, wobei das Lösungsmittel ein Gemisch aus Methanol und Isopropylalkohol ist.

11. Verfahren zur Herstellung einer pharmazeutischen Zusammensetzung, welche eine therapeutisch wirksame Menge von Sertralin-Hydrochlorid Form V und einen pharmazeutisch verträglichen Träger umfaßt, wobei das Verfahren das Verfahren nach einem der vorangegangenen Ansprüche umfaßt.

## Revendications

1. Procédé de fabrication du chlorhydrate de sertraline Forme V comprenant les étapes consistant à :
(a) dissoudre ou mettre en suspension la sertraline base dans un solvant choisi dans le groupe comprenant le méthanol, l'éthanol, l'eau, l'alcool isopropylique, l'hexane et le toluène et leurs mélanges ;
(b) ajouter du chlorure d'hydrogène ou de l'acide chlorhydrique pour réduire le pH de la solution ou de la suspension ; et
(c) isoler le chlorhydrate de sertraline Forme V.

2. Procédé selon la revendication 1 dans lequel le pH de la solution ou de la suspension de la sertraline base et du chlorure d'hydrogène est 0 à 4.

3. Procédé selon la revendication 1 dans lequel le solvant est du méthanol.

4. Procédé selon la revendication 1 dans lequel le solvant est de l'éthanol.

5. Procédé selon la revendication 1 dans lequel le solvant est de l'eau.

6. Procédé selon la revendication 1 dans lequel le solvant est un mélange d'hexane et d'alcool isopropylique.

7. Procédé selon la revendication 1 dans lequel le solvant est un mélange d'alcool isopropylique et d'eau.

8. Procédé selon la revendication 1 dans lequel le solvant est un mélange d'éthanol et d'eau.

9. Procédé selon la revendication 1 dans lequel le solvant est un mélange d'éthanol et d'alcool isopropylique.

10. Procédé selon la revendication 1 dans lequel le solvant est un mélange de méthanol et d'alcool isopropylique.

11. Procédé de préparation d'une composition pharmaceutique comprenant une quantité thérapeutiquement efficace de chlorhydrate de sertraline Forme V et un support pharmaceutiquement acceptable lequel procédé comprend le procédé de l'une quelconque des revendications précédentes.
